# EUROPEAN PATENT APPLICATION

(11) **EP 4 516 325 A1**
(43) Date of publication of application: **05.03.2025**
(21) Application number: 23885910.2
(22) Date of filing: 12.05.2023
(51) Int. Cl.: A61L 2/10, H01J 61/02, H01J 61/073

(54) **UV IRRADIATION DEVICE**

(30) Priority: 01.11.2022 KR 20220143751
(71) Applicant: LG Electronics Inc., Yeongdeungpo-gu Seoul 07336 (KR)
(72) Inventor: PARK, Deokhai, Seoul 08592 (KR); OH, Jinmok, Seoul 08592 (KR); NAMKUNG, Seok, Seoul 08592 (KR)
(74) Representative: Vossius & Partner Patentanwälte Rechtsanwälte mbB
(86) International application number: PCT/KR2023/006510
(87) International publication number: WO 2024/096212

(57) **Abstract**

An ultraviolet irradiation device is provided. The ultraviolet irradiation device according to an aspect of the present disclosure includes a housing, a plurality of electrodes configured to extend in a first direction in the housing and be spaced apart in a second direction perpendicular to the first direction, a plurality of ultraviolet lamps configured to emit ultraviolet light with a wavelength of 190 nm or more and 225 nm or less, extend in the second direction on the plurality of electrodes, and be spaced apart in the first direction, and a reflective member disposed between the plurality of ultraviolet lamps and the plurality of electrodes.

## Description

### [Technical Field]

The present disclosure relates to an ultraviolet irradiation device. More specifically, the present disclosure relates to an ultraviolet irradiation device irradiating UV-C ultraviolet light.

### [Background Art]

Pathogens such as bacteria and fungi are everywhere, including on doorknobs, telephones, television remote controls, public restrooms, countertops, sidewalks, and the air.

In general, ultraviolet lamps are used in various fields to sterilize bacteria and fungi by generating ultraviolet light.

The ultraviolet lamp can be suitably used with easy operation at the necessary time by using a lamp method. There is an advantage in that the installation cost and the maintenance cost for installing the ultraviolet lamp are low. In addition, the ultraviolet light generated by the ultraviolet lamp rarely changes and thus continuously has the same sterilizing power.

The ultraviolet lamp generates ultraviolet (UV) light with various wavelengths based on a material provided inside the lamp. For example, UV-A (wavelength of 400 nm to 315 nm), UV-B (wavelength of 315 nm to 280 nm), UV-C (wavelength of 280 nm to 110 nm), and the like may be generated.

Among them, ultraviolet light with a wavelength of 253.7 nm at the wavelength corresponding to UV-C has the best sterilizing power. When UV-C is irradiated to bacteria, fungi, etc., bacteria and fungi die because their DNA is damaged. That is, ultraviolet light has an effective sterilizing power against various bacteria by damaging DNA of living organisms.

Therefore, sterilization using the ultraviolet lamp is more efficient than sterilization using heat, chemicals, ozone, or radiation.

When ultraviolet light with a wavelength of 253.7 nm is irradiated to the human body, the ultraviolet light may adversely affect the human body.

### [Disclosure]

### [Technical Problem]

An object of the present disclosure is to provide an ultraviolet irradiation device that can improve an amount of light emitted upward from an ultraviolet lamp.

Another object of the present disclosure is to provide an ultraviolet irradiation device that can improve an amount of light without a separate process such as cutting or bonding.

Another object of the present disclosure is to provide an ultraviolet irradiation device that can improve contact between a reflective member and a plurality of electrodes.

Another object of the present disclosure is to provide an ultraviolet irradiation device that can improve an amount of light of an ultraviolet lamp in a space where a plurality of electrodes is not disposed.

### [Technical Solution]

In order to achieve the above-described objects, in one aspect of the present disclosure, there is provided an ultraviolet irradiation device comprising a housing, a plurality of electrodes configured to extend in a first direction in the housing and be spaced apart in a second direction perpendicular to the first direction, a plurality of ultraviolet lamps configured to emit ultraviolet light with a wavelength of 190 nm or more and 225 nm or less, extend in the second direction on the plurality of electrodes, and be spaced apart in the first direction, and a reflective member disposed between the plurality of ultraviolet lamps and the plurality of electrodes.

Through this, the present disclosure can improve an amount of light emitted upward from the ultraviolet lamps.

The reflective member may be formed in a film shape and may cover entire upper surfaces of the plurality of electrodes.

Through this, the present disclosure can improve an amount of light of the ultraviolet irradiation device without a separate process such as cutting or bonding.

The plurality of electrodes may include a first electrode block formed of a first electrode and a second electrode block that is formed of a second electrode and is spaced apart from the first electrode block in the second direction. The reflective member may include a first reflective member disposed on the first electrode block and a second reflective member disposed on the second electrode block.

A thickness of the reflective member may be 70 µm or more and 130 µm or less.

A width where each of the plurality of ultraviolet lamps is in contact with the reflective member may be 190 ° or more and 280 ° or less.

Through this, the present disclosure can improve the amount of light emitted upward from the ultraviolet lamps.

Each of the plurality of electrodes may include a tapered surface in contact with each of the plurality of ultraviolet lamps. The reflective member may be disposed below an area where the tapered surfaces are in contact with the plurality of ultraviolet lamps, and may extend in the second direction.

The reflective member may include a plurality of reflective members that extends in the second direction and is spaced apart from each other in the first direction. Based on being viewed from above, an area of each of the plurality of reflective members may be larger than an area of each of the plurality of ultraviolet lamps.

The plurality of electrodes may be formed of an aluminum material, and the reflective member may be formed of a polytetrafluoroethylene (PTFE) material.

Through this, the present disclosure can improve contact between the reflective member and the plurality of electrodes.

The ultraviolet irradiation device may further comprise an additional reflective member disposed on the housing in a space between the plurality of electrodes and configured to face the plurality of ultraviolet lamps.

Through this, the present disclosure can improve an amount of light of the ultraviolet lamps in a space in which the plurality of electrodes is not disclosed.

The ultraviolet irradiation device may further comprise an optical filter coupled to the housing and disposed on the plurality of ultraviolet lamps.

In order to achieve the above-described objects, in one aspect of the present disclosure, there is provided an ultraviolet irradiation device comprising a housing, a plurality of electrodes spaced apart in a first direction in the housing and configured to extend in a second direction perpendicular to the first direction, a plurality of ultraviolet lamps configured to emit ultraviolet light with a wavelength of 190 nm or more and 225 nm or less, extend in the second direction between the plurality of electrodes, and be spaced apart in the first direction, and a reflective member disposed between the plurality of ultraviolet lamps and the plurality of electrodes.

Through this, the present disclosure can improve an amount of light emitted upward from the ultraviolet lamps.

The reflective member may be formed in a film shape and may cover entire upper surfaces of the plurality of electrodes.

Through this, the present disclosure can improve an amount of light of the ultraviolet irradiation device without a separate process such as cutting or bonding.

The plurality of electrodes may include a first electrode block formed of a first electrode and a second electrode block that is formed of a second electrode and is spaced apart from the first electrode block in the first direction. The reflective member may include a plurality of reflective members that is disposed between the first electrode block and the second electrode block and is spaced apart in the first direction.

A thickness of the reflective member may be 70 µm or more and 130 µm or less.

A width where each of the plurality of ultraviolet lamps is in contact with the reflective member may be 190 ° or more and 280 ° or less.

Through this, the present disclosure can improve an amount of light emitted upward from the ultraviolet lamps.

The plurality of electrodes may be formed of an aluminum material, and the reflective member may be formed of a polytetrafluoroethylene (PTFE) material.

Through this, the present disclosure can improve contact between the reflective member and the plurality of electrodes.

The reflective member may include a plurality of reflective members that extends in the second direction and is spaced apart from each other in the first direction. Based on being viewed from above, an area of each of the plurality of reflective members may be larger than an area of each of the plurality of ultraviolet lamps.

### [Advantageous Effects]

The present disclosure can provide an ultraviolet irradiation device that can improve an amount of light emitted upward from an ultraviolet lamp.

The present disclosure can provide an ultraviolet irradiation device that can improve an amount of light without a separate process such as cutting or bonding.

The present disclosure can provide an ultraviolet irradiation device that can improve contact between a reflective member and a plurality of electrodes.

The present disclosure can provide an ultraviolet irradiation device that can improve an amount of light of an ultraviolet lamp in a space where a plurality of electrodes is not disposed.

### [Description of Drawings]

FIG. 1 is a perspective view of an ultraviolet irradiation device according to an embodiment of the present disclosure.
FIG. 2 is an exploded perspective view of an ultraviolet irradiation device according to an embodiment of the present disclosure.
FIG. 3 is a cross-sectional view of an ultraviolet irradiation device according to an embodiment of the present disclosure.
FIGS. 4 to 8 are modifications of an ultraviolet irradiation device according to an embodiment of the present disclosure.
FIG. 9 illustrates that a reflective member is removed from the ultraviolet irradiation device of FIG. 3.
FIG. 10 illustrates an amount of light of the ultraviolet irradiation device of FIGS. 4 to 8.
FIG. 11 illustrates an ultraviolet lamp and a reflective member of an ultraviolet irradiation device according to an embodiment of the present disclosure.
FIG. 12 illustrates an amount of light depending on a thickness of a reflective member of an ultraviolet irradiation device according to an embodiment of the present disclosure.
FIG. 13 illustrates an amount of light depending on a width of a reflective member of an ultraviolet irradiation device according to an embodiment of the present disclosure.
FIG. 14 is a perspective view of an ultraviolet irradiation device according to another embodiment of the present disclosure.
FIG. 15 is an exploded perspective view of an ultraviolet irradiation device according to another embodiment of the present disclosure.

### [Mode for Invention]

Reference will now be made in detail to embodiments of the present disclosure, examples of which are illustrated in the accompanying drawings. Wherever possible, the same reference numbers will be used throughout the drawings to refer to the same or like parts.

It should be understood that when a component is described as being "connected to" or "coupled to" other component, the component may be directly connected or coupled to the other component or intervening component(s) may be present.

It will be noted that a detailed description of known arts will be omitted if it is determined that the detailed description of the known arts can obscure embodiments of the present disclosure. The accompanying drawings are used to help easily understand various technical features and it should be understood that embodiments presented herein are not limited by the accompanying drawings. As such, the present disclosure should be understood to extend to any alterations, equivalents and substitutes in addition to those which are particularly set out in the accompanying drawings.

In addition, a term of "disclosure" may be replaced by terms such as document, specification, description, etc.

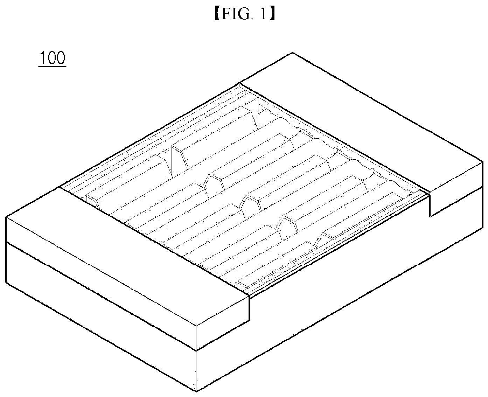

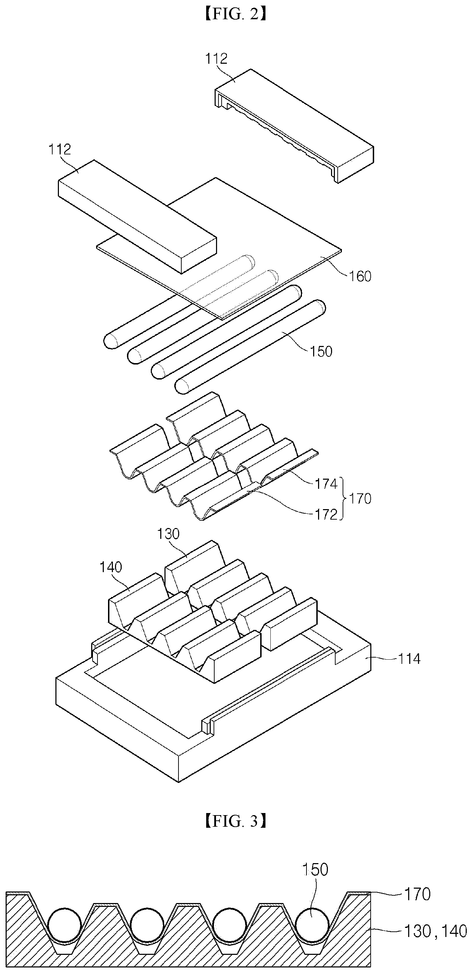

FIG. 1 is a perspective view of an ultraviolet irradiation device according to an embodiment of the present disclosure. FIG. 2 is an exploded perspective view of an ultraviolet irradiation device according to an embodiment of the present disclosure. FIG. 3 is a cross-sectional view of an ultraviolet irradiation device according to an embodiment of the present.

Referring to FIGS. 1 to 3, an ultraviolet irradiation device 100 according to an embodiment of the present disclosure may include a housing 110, electrodes 130 and 140, an ultraviolet lamp 150, an optical filter 160, and a reflective member 170, but may be implemented except for some of these components and may not exclude other additional components.

The ultraviolet irradiation device 100 according to an embodiment of the present disclosure may be interpreted to mean a device generating UV-C, but is not limited thereto. The ultraviolet irradiation device 100 may be carried portably or installed in a certain location. The ultraviolet irradiation device 100 may emit ultraviolet light with a wavelength that is harmless to the human body and has sterilizing power. The ultraviolet irradiation device 100 may be formed in a hexahedral shape as a whole.

The housing 110 may form an appearance of the ultraviolet irradiation device 100. The housing 110 may be formed in a hexahedral shape as a whole. The electrodes 130 and 140, the ultraviolet lamp 150, the optical filter 160, and the reflective member 170 may be disposed in the housing 110.

The housing 110 may include an upper housing 112 and a lower housing 114.

A mounting space in which the electrodes 130 and 140 are mounted may be formed in the lower housing 114. The upper housing 112 may be coupled to the lower housing 114. The upper housing 112 may fix the ultraviolet lamp 150 and the optical filter 160 while being coupled to the lower housing 114.

The electrodes 130 and 140 may extend in a first direction. The electrodes 130 and 140 may include a plurality of electrodes 130 and 140 that are spaced apart from each other in a second direction.

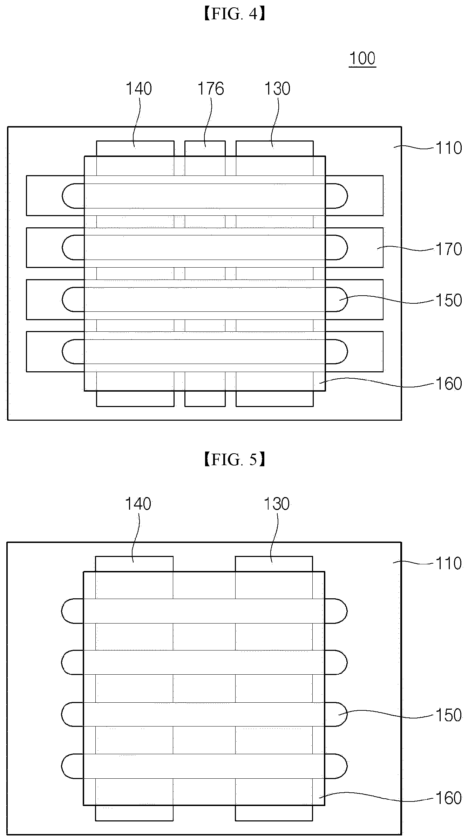

In an embodiment of the present disclosure, the first direction may be interpreted to mean an up-down direction based on FIG. 4, and the second direction may be interpreted to mean a left-right direction based on FIG. 4.

The plurality of electrodes 130 and 140 may include a first electrode block 130 formed of a first electrode and a second electrode block 140 that is formed of a second electrode and is spaced apart from the first electrode block 130 in the second direction. The first electrode block 130 and the second electrode block 140 may be formed in a shape corresponding to each other.

The electrodes 130 and 140 may include a tapered surface on which the ultraviolet lamp 150 is mounted. The ultraviolet lamp 150 may be in contact with the tapered surfaces of the electrodes 130 and 140.

The ultraviolet lamp 150 may be disposed in the housing 110. The ultraviolet lamp 150 may be formed in a cylindrical shape. The ultraviolet lamp 150 may be formed in a tube shape. The ultraviolet lamp 150 may extend in the second direction. The ultraviolet lamp 150 may be disposed on the electrodes 130 and 140. The ultraviolet lamp 150 may be disposed on the tapered surfaces of the electrodes 130 and 140. An outer circumferential surface of the ultraviolet lamp 150 may be in contact with the tapered surfaces of the electrodes 130 and 140. A central area of the ultraviolet lamp 150 may be mounted on the tapered surfaces of the electrodes 130 and 140, and both ends of the ultraviolet lamp 150 may be fixed by the upper housing 112. The ultraviolet lamp 150 may include a plurality of ultraviolet lamps 150 spaced apart from each other in the first direction.

The ultraviolet lamp 150 may generate UV-C with a wavelength of 110 nm or more and 180 nm or less. Specifically, the ultraviolet lamp 150 may emit ultraviolet light with a wavelength of 190 nm or more and 225 nm or less. More specifically, the ultraviolet lamp 150 may emit ultraviolet light with a wavelength of 222 nm. The ultraviolet lamp 150 may be an excimer lamp in which a light emitting gas including KrCL, KrBr, and ArF is sealed. As a result, the ultraviolet lamp 150 can eradicate DNA of bacteria or fungi, which is harmless to the human body.

The optical filter 160 may be coupled to the housing 110. The optical filter 160 may be disposed on the ultraviolet lamp 150. Specifically, the optical filter 160 may be mounted on the lower housing 114 and fixed by the lower housing 114. The optical filter 160 may not transmit ultraviolet light with a wavelength of 240 nm or more and 300 nm or less. The optical filter 160 may substantially transmit ultraviolet light with a wavelength of 190 nm or more and 225 nm or less. As a result, the optical filter 160 can prevents ultraviolet light with wavelengths affecting the human body from being exposed to the outside.

The reflective member 170 may be disposed in the housing 110. The reflective member 170 may be disposed between the ultraviolet lamp 150 and the plurality of electrodes 130 and 140. The reflective member 170 may be formed in a thin film shape. The reflective member 170 may upward reflect light emitted downward from the ultraviolet lamp 150. As a result, the reflective member 170 can improve an amount of light emitted upward from the ultraviolet lamp 150.

The reflective member 170 may be formed of a flexible polytetrafluoroethylene (PTFE) material. Specifically, the reflective member 170 may be formed of a flexible material containing 90% or more of PTFE. When the plurality of electrodes 130 and 140 is formed of an aluminum material, unevenness in a contact surface between the plurality of electrodes 130 and 140 and the ultraviolet lamp 150 may occur due to uneven distribution of an outer diameter or straightness of the ultraviolet lamp 150. Since the reflective member 170 is formed of the PTFE material, the contact between the plurality of electrodes 130 and 140 and the ultraviolet lamp 150 can be improved.

The reflective member 170 may be formed in a film shape and may cover entire upper surfaces of the plurality of electrodes 130 and 140. When the reflective member 170 has a thin film shape and covers the upper surfaces of the electrodes 130 and 140 as illustrated in FIG. 2, the reflective member 170 may be deformed into the same shape as the upper surfaces of the electrodes 130 and 140. As a result, the reflective member 170 can improve an amount of light of the ultraviolet irradiation device 100 without a separate process such as cutting or bonding.

A separate adhesive may be provided between the film-shaped reflective member 170 and the plurality of electrodes 130 and 140. In this case, structural stability of the ultraviolet irradiation device 100 can be improved, but a reflectance of the reflective member 170 may be reduced.

Alternatively, the reflective member 170 may be formed by spraying PTFE powder on the upper surfaces of the plurality of electrodes 130 and 140 and performing drying and heat treatment on it. In this case, the reflectance of the reflective member 170 may be reduced due to an increase in other additives.

The reflective member 170 may include a first reflective member 174 disposed on the first electrode block 130 and a second reflective member 172 disposed on the second electrode block 140. In this case, each of the first reflective member 174 and the second reflective member 172 may be formed in a thin and flexible film shape. When the first reflective member 174 is disposed on the first electrode block 130, the first reflective member 174 may be deformed into a shape corresponding to the upper surface of the first electrode block 130, and when the second reflective member 172 is disposed on the second electrode block 140, the second reflective member 172 may be deformed into a shape corresponding to the upper surface of the second electrode block 140.

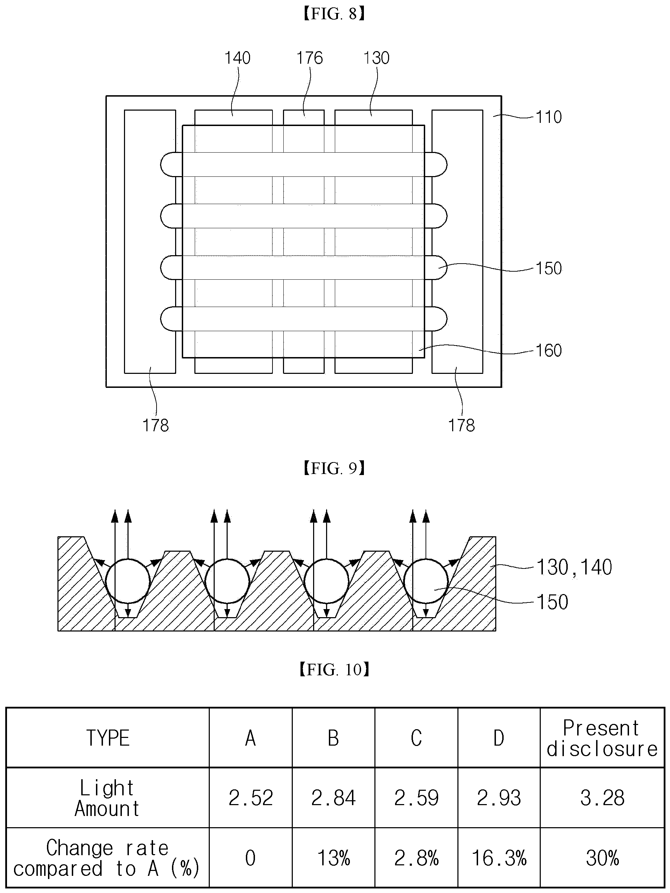

FIGS. 4 to 8 are modifications of an ultraviolet irradiation device according to an embodiment of the present disclosure. FIG. 9 illustrates that a reflective member is removed from the ultraviolet irradiation device of FIG. 3. FIG. 10 illustrates an amount of light of the ultraviolet irradiation device of FIGS. 4 to 8.

Referring to FIG. 4, the reflective member 170 of the ultraviolet irradiation device 100 according to an embodiment of the present disclosure may include a plurality of reflective members 170 that extends in the second direction and is spaced apart from each other in the first direction. When viewed from above, the area of each of the plurality of reflective members 170 may be larger than the area of each of the plurality of ultraviolet lamps 150. Through this, since ultraviolet light directed downward from the ultraviolet lamps 150 is reflected and directed upward, the amount of light directed upward from the ultraviolet lamps 150 can be improved.

Further, the ultraviolet irradiation device 100 may include an additional reflective member 176 that is disposed on the housing 110 in a space between the plurality of electrodes 130 and 140 and faces the plurality of ultraviolet lamps 150. The additional reflective member 176 may be disposed on the lower housing 114. The additional reflective member 176 may be disposed between the first electrode block 130 and the second electrode block 140. The additional reflective member 176 may extend in the first direction. As a result, the additional reflective member 176 can improve the amount of light directed upward from the ultraviolet lamps 150 in a space in which the plurality of electrodes 130 and 140 are not disposed.

Referring to FIG. 5, an ultraviolet irradiation device A according to an embodiment of the present disclosure may not include the reflective member 170 and the additional reflective member 176.

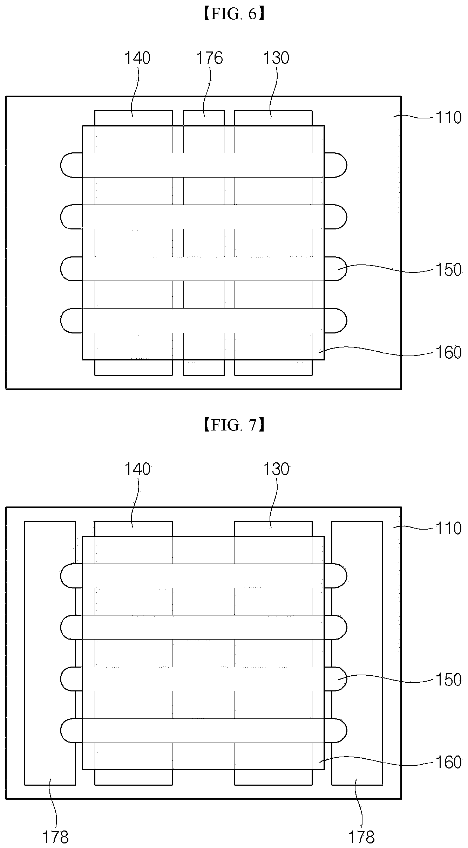

Referring to FIG. 6, an ultraviolet irradiation device B according to an embodiment of the present disclosure may be configured to add the additional reflective member 176 to the ultraviolet irradiation device A of FIG. 5.

Referring to FIG. 7, an ultraviolet irradiation device C according to an embodiment of the present disclosure may be configured to add both reflective members 178 to the ultraviolet irradiation device A of FIG. 5. The both reflective members 178 may extend from both ends of the plurality of ultraviolet lamps 150 in the first direction. The both reflective members 178 may be disposed in the lower housing 114.

Referring to FIG. 8, an ultraviolet irradiation device D according to an embodiment of the present disclosure may be configured to add the additional reflective member 176 and both reflective members 178 to the ultraviolet irradiation device A of FIG. 5.

Referring to FIG. 9, when the reflective member 170 is not disposed between the plurality of electrodes 130 and 140 and the plurality of ultraviolet lamps 150, light emitted downward from the plurality of ultraviolet lamps 150 is reflected upward from the plurality of electrodes 130 and 140. Hence, light leakage occurs. Therefore, as illustrated in FIG. 3, the reflective member 170 that is in direct contact with the plurality of ultraviolet lamps 150 can reduce the leakage of light and improve an amount of light emitted upward from the ultraviolet irradiation device 100.

Referring to FIG. 10, it can be seen that the amount of light in the ultraviolet irradiation device 100 based on FIG. 4 is increased by about 30% compared to the amount of light in the ultraviolet irradiation device A based on FIG. 5. It can also be seen that the amount of light in the ultraviolet irradiation device 100 based on FIG. 4 is increased compared to the amount of light in the ultraviolet irradiation devices B, C, and D based on FIGS. 6 to 8. That is, when viewed from above, the area of each of the plurality of reflective members 170 may be larger than the area of each of the plurality of ultraviolet lamps 150, and the amount of light of the ultraviolet irradiation device 100 can be significantly improved as the additional reflective member 176 is provided.

Alternatively, the plurality of reflective members 170 may be disposed only below an area where the tapered surfaces of the plurality of electrodes 130 and 140 are in contact with the plurality of ultraviolet lamps 150. In this case, since light leaked downward from the plurality of ultraviolet lamps 150 is reflected upward while improving the ease of manufacturing, the amount of light of the ultraviolet irradiation device 100 can be improved.

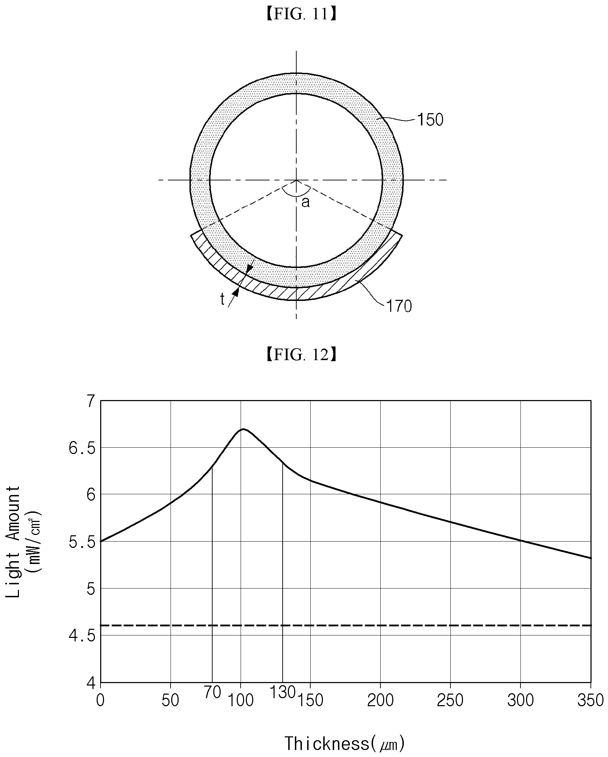

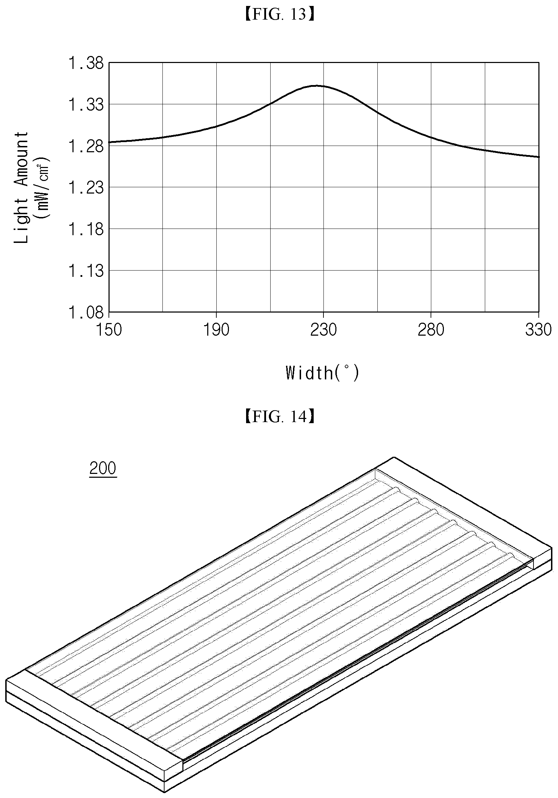

FIG. 11 illustrates an ultraviolet lamp and a reflective member of an ultraviolet irradiation device according to an embodiment of the present disclosure. FIG. 12 illustrates an amount of light depending on a thickness of a reflective member of an ultraviolet irradiation device according to an embodiment of the present disclosure. FIG. 13 illustrates an amount of light depending on a width of a reflective member of an ultraviolet irradiation device according to an embodiment of the present disclosure.

Referring to FIG. 11, a thickness t and a width 'a' of the reflective member 170 in contact with the ultraviolet lamp 150 of the ultraviolet irradiation device 100 are described. Here, the thickness t of the reflective member 170 may refer to a radial length of the ultraviolet lamp 150, and the width 'a' of the reflective member 170 may refer to a circumferential angle of the ultraviolet lamp 15.

Referring to FIG. 12, the thickness t of the reflective member 170 may be 70 µm or more and 130 µm or less. It can be seen that when the thickness t of the reflective member 170 is less than 70 µm or exceeds 130 µm, an increase rate of the amount of light is significantly reduced. Therefore, when the thickness t of the reflective member 170 is 70 µm or more and 130 µm or less, the amount of light of the ultraviolet irradiation device 100 can be maximized.

Referring to FIG. 13, the width 'a' where the ultraviolet lamp 150 is in contact with the reflective member 170 may be 190 ° or more and 280 ° or less. It can be seen that when the width 'a' of the reflective member 170 is less than 190 ° or exceeds 280 °, an increase rate of the amount of light is significantly reduced. Therefore, when the width 'a' of the reflective member 170 is 190 ° or more and 280 ° or less, the amount of light of the ultraviolet irradiation device 100 can be maximized.

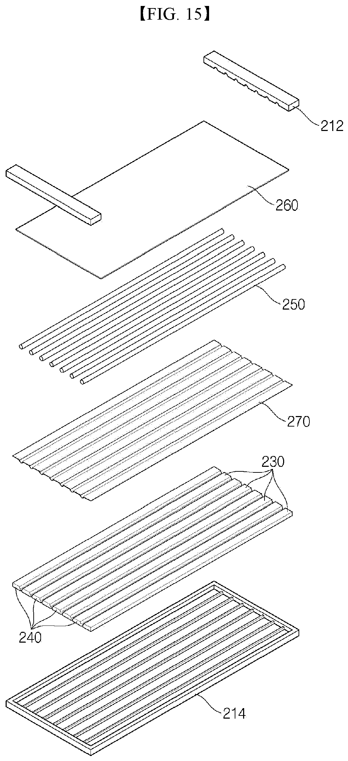

FIG. 14 is a perspective view of an ultraviolet irradiation device according to another embodiment of the present disclosure. FIG. 15 is an exploded perspective view of an ultraviolet irradiation device according to another embodiment of the present disclosure.

Referring to FIGS. 14 and 15, an ultraviolet irradiation device 200 according to another embodiment of the present disclosure may include a housing 210, a plurality of electrodes 230 and 240, a plurality of ultraviolet lamps 250, an optical filter 260, and a reflective member 270, but may be implemented except for some of these components and may not exclude other additional components.

The detailed configuration of the ultraviolet irradiation device 200 according to another embodiment of the present disclosure not described below can be understood to be the same as the detailed configuration of the ultraviolet irradiation device 100 according to an embodiment of the present disclosure.

The plurality of electrodes 230 and 240 may be spaced apart from each other in a first direction. The plurality of electrodes 230 and 240 may extend in a second direction. That is, the extending direction of the plurality of electrodes 230 and 240 of the ultraviolet irradiation device 200 according to another embodiment of the present disclosure may be different from the extending direction of the plurality of electrodes 130 and 140 of the ultraviolet irradiation device 100 according to an embodiment of the present disclosure.

The plurality of electrodes 230 and 240 may include first electrode blocks 230 extending in the second direction and second electrode blocks 240 that extend in the second direction and are spaced apart from the first electrode blocks 230 in the first direction. The plurality of electrodes 230 and 240 may be configured such that the first electrode blocks 230 and the second electrode blocks 240 are alternately arranged. The ultraviolet lamps 250 extending in the second direction may be disposed on tapered surfaces of the first electrode blocks 230 and the second electrode blocks 240.

The ultraviolet lamp 250 may include the plurality of ultraviolet lamps 250 that extend in the second direction between the plurality of electrodes 230 and 240 and are spaced apart in the first direction.

The reflective member 270 may be disposed between the plurality of ultraviolet lamps 250 and the plurality of electrodes 230 and 240. Through this, the reflective member 270 can improve an amount of light emitted upward from the ultraviolet lamps 250.

The reflective member 270 may be formed in a film shape and may cover entire upper surfaces of the plurality of electrodes 230 and 240. Through this, the reflective member 270 can improve an amount of light of the ultraviolet irradiation device 200 without a separate process such as cutting or bonding.

The reflective member 270 may include a plurality of reflective members 270 that are disposed between the first electrode block 230 and the second electrode block 240 and are spaced apart in the first direction.

In an area where the reflective member 270 is in contact with the ultraviolet lamp 250, a thickness t of the reflective member 270 may be 70 µm or more and 130 µm or less. In the area where the reflective member 270 is in contact with the ultraviolet lamp 250, a width 'a' of the reflective member 270 may be 190 ° or more and 280 ° or less. As a result, the amount of light emitted upward from the ultraviolet lamp 250 can be improved.

The plurality of electrodes 230 and 240 may be formed of an aluminum material, and the reflective member 270 may be formed of a PTFE material. As a result, the contact between the reflective member 270 and the plurality of electrodes 230 and 240 and the contact between the reflective member 270 and the ultraviolet lamp 250 can be improved.

The reflective member 270 may include a plurality of reflective members 270 that extend in the second direction and are spaced apart in the first direction. In this case, when viewed from above, an area of each of the plurality of reflective members 270 may be larger than an area of each of the plurality of ultraviolet lamps 250. Through this, the amount of light directed upward from the ultraviolet irradiation device 200 can be improved.

Some embodiments or other embodiments of the present disclosure described above are not exclusive or distinct from each other. Some embodiments or other embodiments of the present disclosure described above can be used together or combined in configuration or function.

For example, configuration "A" described in an embodiment and/or the drawings and configuration "B" described in another embodiment and/or the drawings can be combined with each other. That is, even if the combination between the configurations is not directly described, the combination is possible except in cases where it is described that it is impossible to combine.

The above detailed description is merely an example and is not to be considered as limiting the present disclosure. The scope of the present disclosure should be determined by rational interpretation of the appended claims, and all variations within the equivalent scope of the present disclosure are included in the scope of the present disclosure.

## Claims

1. An ultraviolet irradiation device comprising:
a housing;
a plurality of electrodes configured to extend in a first direction in the housing and be spaced apart in a second direction perpendicular to the first direction;
a plurality of ultraviolet lamps configured to emit ultraviolet light with a wavelength of 190 nm or more and 225 nm or less, extend in the second direction on the plurality of electrodes, and be spaced apart in the first direction; and
a reflective member disposed between the plurality of ultraviolet lamps and the plurality of electrodes.

2. The ultraviolet irradiation device of claim 1, wherein the reflective member is formed in a film shape and covers entire upper surfaces of the plurality of electrodes.

3. The ultraviolet irradiation device of claim 1, wherein the plurality of electrodes includes a first electrode block formed of a first electrode and a second electrode block that is formed of a second electrode and is spaced apart from the first electrode block in the second direction, and
wherein the reflective member includes a first reflective member disposed on the first electrode block and a second reflective member disposed on the second electrode block.

4. The ultraviolet irradiation device of claim 1, wherein a thickness of the reflective member is 70 µm or more and 130 µm or less.

5. The ultraviolet irradiation device of claim 1, wherein a width where each of the plurality of ultraviolet lamps is in contact with the reflective member is 190 ° or more and 280 ° or less.

6. The ultraviolet irradiation device of claim 1, wherein each of the plurality of electrodes includes a tapered surface in contact with each of the plurality of ultraviolet lamps, and
wherein the reflective member is disposed below an area where the tapered surfaces are in contact with the plurality of ultraviolet lamps, and extends in the second direction.

7. The ultraviolet irradiation device of claim 1, wherein the reflective member includes a plurality of reflective members that extends in the second direction and is spaced apart from each other in the first direction, and
wherein, based on being viewed from above, an area of each of the plurality of reflective members is larger than an area of each of the plurality of ultraviolet lamps.

8. The ultraviolet irradiation device of claim 1, wherein the plurality of electrodes is formed of an aluminum material, and
wherein the reflective member is formed of a polytetrafluoroethylene (PTFE) material.

9. The ultraviolet irradiation device of claim 1, further comprising:
an additional reflective member disposed on the housing in a space between the plurality of electrodes and configured to face the plurality of ultraviolet lamps.

10. The ultraviolet irradiation device of claim 1, further comprising:
an optical filter coupled to the housing and disposed on the plurality of ultraviolet lamps.

11. An ultraviolet irradiation device comprising:
a housing;
a plurality of electrodes spaced apart in a first direction in the housing and configured to extend in a second direction perpendicular to the first direction;
a plurality of ultraviolet lamps configured to emit ultraviolet light with a wavelength of 190 nm or more and 225 nm or less, extend in the second direction between the plurality of electrodes, and be spaced apart in the first direction; and
a reflective member disposed between the plurality of ultraviolet lamps and the plurality of electrodes.

12. The ultraviolet irradiation device of claim 11, wherein the reflective member is formed in a film shape and covers entire upper surfaces of the plurality of electrodes.

13. The ultraviolet irradiation device of claim 11, wherein the plurality of electrodes includes a first electrode block formed of a first electrode and a second electrode block that is formed of a second electrode and is spaced apart from the first electrode block in the first direction, and
wherein the reflective member includes a plurality of reflective members that is disposed between the first electrode block and the second electrode block and is spaced apart in the first direction.

14. The ultraviolet irradiation device of claim 11, wherein a thickness of the reflective member is 70 µm or more and 130 µm or less.

15. The ultraviolet irradiation device of claim 11, wherein a width where each of the plurality of ultraviolet lamps is in contact with the reflective member is 190 ° or more and 280 ° or less.

16. The ultraviolet irradiation device of claim 11, wherein the plurality of electrodes is formed of an aluminum material, and
wherein the reflective member is formed of a polytetrafluoroethylene (PTFE) material.

17. The ultraviolet irradiation device of claim 11, wherein the reflective member includes a plurality of reflective members that extends in the second direction and is spaced apart from each other in the first direction, and
wherein, based on being viewed from above, an area of each of the plurality of reflective members is larger than an area of each of the plurality of ultraviolet lamps.
